(19) Europäisches Patentamt
European Patent Office
Office européen des brevets

(11) **EP 4 599 773 A1**

(12) **EUROPEAN PATENT APPLICATION**

(43) Date of publication:
**13.08.2025 Bulletin 2025/33**

(21) Application number: 24305205.7

(22) Date of filing: **07.02.2024**

(51) International Patent Classification (IPC):
**A61B 8/08** $^{(2006.01)}$    **A61B 8/00** $^{(2006.01)}$

(52) Cooperative Patent Classification (CPC):
**A61B 8/0891; A61B 8/461; A61B 8/483;**
**A61B 8/5207; G06T 5/94**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **MORY, Benoit**
  **5656 Eindhoven (NL)**
• **FLORKOW, Mateusz**
  **5656 Eindhoven (NL)**
• **SOMPHONE, Oudom**
  **5656 Eindhoven (NL)**
• **LOUPAS, Thanasis**
  **5656 Eindhoven (NL)**

(74) Representative: **Philips Intellectual Property &**
**Standards**
**High Tech Campus 52**
**5656 AG Eindhoven (NL)**

(54) **SCALE-ADAPTIVE ULTRASOUND SURFACE SHADING**

(57)     An ultrasound system (210) includes an interface, a memory (151), and a processor (152). The interface receives two-dimensional ultrasound images. The memory (151) stores instructions and the two-dimensional ultrasound images. The processor (152) executes the instructions. When executed by the processor (152), the instructions cause the ultrasound system (210) to: for each of a plurality of pixels of each two-dimensional ultrasound image, identify an optimal scale for applying surface shading based on a size of at least one vessel in the two-dimensional ultrasound image; apply surface shading to at least one first pixel based on identifying the optimal scale for the at least one first pixel; and apply surface shading to at least one second pixel based on identifying the second scale as an optimal scale for the at least one second pixel.

FIG.1

## Description

FIELD OF THE INVENTION

The invention relates to imaging visualization. In particular to two-dimensional to three-dimensional visualization conversion in ultrasound image visualization.

BACKGROUND OF THE INVENTION

An ultrasound visualization technique known as flow viewer has recently been developed to provide three-dimensional-like rendering of two-dimensional ultrasound flow imaging data. The flow viewer visualization technique is applicable to all ultrasound flow imaging modes including color Doppler, power Doppler (also known as CPA), directional power Doppler (also known as dCPA), microflow imaging (also known as MFI), and high-definition MFI (also known as MFI-HD). The flow viewer visualization technique can be applied both during real-time two-dimensional ultrasound imaging as well as in post-processing. The basis of the flow viewer visualization technique is to apply a known Blinn-Phong reflection model for three-dimensional surfaces to two-dimensional ultrasound images. More specifically, the flow viewer visualization algorithm requires two input two-dimensional ultrasound images including a luminance image and a red/green/blue (RGB) image. The two-dimensional luminance image is used to create lighting effects for the two-dimensional RGB image. As an example, the two-dimensional RGB image may comprise a color-coded Doppler velocity map. The two-dimensional luminance image may comprise the power component of the Doppler signal which is used (a) indirectly, to determine if a pixel corresponds to flow or not during color Doppler flow imaging; and (b) directly, during power Doppler flow imaging (e.g., while in CPA, dCPA, MFI, MFI-HD).

The two-dimensional luminance image used in the flow viewer visualization technique may be processed through a low-pass gradient filter to produce a three-dimensional gradient vector field which is used to apply surface shading to the two-dimensional RGB image. The generated surface is defined by its three-dimensional normal vector, corresponding to the smooth three-dimensional gradient vector field. A smooth three-dimensional surface for the three-dimensional gradient vector field is generated by convolving the two-dimensional luminance image with a Gaussian kernel to provide the three-dimensional gradient vector field used to apply surface shading to the two-dimensional RGB image. The scale of the Gaussian kernel is a parameter that controls the smoothness of the three-dimensional surface of the gradient vector field and the size of the details that will be enhanced by lighting effects. The size of the Gaussian kernel is determined by a scale parameter and reflects the amount of smoothing applied. The gradient vector field has three components including Gx, Gy, and 1. Gx is the horizontal derivative of the pre-filtered two-dimensional luminance image and Gy is the vertical derivative of the pre-filtered two-dimensional luminance image. The Gx and Gy components of the smooth three-dimensional surface of the gradient vector field are multiplied by an Altitude parameter which controls the "peakiness" of the output image. The generated three-dimensional surface of the gradient vector field is then fed to a surface shading module that generates a diffuse map and a specular map, which are combined with the input RGB image to generate the final three-dimensional-like flow viewer output image.

In the surface shading module, the three-dimensional vector normal to the surface $N(x,y)$ is calculated at each pixel x,y as $[Gx; Gy; 1]$. A diffuse component is then obtained as the dot product of $N(x,y)$ and the light source vector L. $H(x,y)$ is then calculated at each pixel x,y as the half-angle vector between the light source direction L and viewer direction V. The specular component $S(x,y)$ is then obtained by raising the dot product of $N(x,y)$ and $H(x,y)$ to the power of the shininess parameter. The output RGB image $Out(x,y)$ is then calculated at each pixel x,y from the input RGB image $In(x,y)$ according to $Out(x,y) = [AmbientC + DiffuseC*D(x,y)]*In(x,y) + SpecularC*S(x,y)$, where AmbientC, DiffuseC and SpecularC are each coefficient parameters. The AmbientC coefficient parameters are from the input RGB image, whereas the DiffuseC coefficient parameters and SpecularC coefficient parameters are from the diffuse map and the specular map generated from the three-dimensional surface of the gradient vector field.

Two main shortcomings of the above technique arise from the arbitrary choice of a single Gaussian kernel scale for the gradient filter. A first shortcoming is that the scale is not set according to the size of vessels present in the RGB image, and this prevents optimizing results in terms of visual separation. A second shortcoming is that the technique does not work well when multiple vessels sizes are present in the same image (e.g., a large vein close to a small artery), so that users must accept a suboptimal compromise.

SUMMARY OF THE INVENTION

It is inter alia, an object of the invention to provide an improved visualization. This is achieved by producing a virtual lighting effect on the two-dimensional images, wherein the pixels of a two-dimensional image are modified to generate a three-dimensional visualization effect.

The invention is defined by the independent claims. Advantageous embodiments are defined by the dependent claims.

According to an aspect of the present disclosure, an ultrasound system includes an interface, a memory, and a processor. The interface receives two-dimensional ultrasound images. The memory stores instructions and the two-dimensional ultrasound images. The processor executes the instructions. When executed by the processor, the instructions cause the ultrasound system to: for each of a plurality of pixels of each two-dimensional ultrasound image, identify an optimal scale for applying surface shading based on a size of at least one vessel in the two-dimensional ultrasound image; apply surface shading to at least one first pixel based on identifying the optimal scale for the at least one first pixel; and apply surface shading to at least one second pixel based on identifying the optimal scale for the at least one second pixel.

According to another aspect of the present disclosure, a method for applying virtual lighting to ultrasound images includes receiving two-dimensional ultrasound images; for each of a plurality of pixels of each two-dimensional ultrasound image, identifying an optimal scale for applying surface shading based on a size of at least one vessel in the two-dimensional ultrasound image; applying surface shading to at least one first pixel based on identifying the optimal scale for the at least one first pixel; and applying surface shading to at least one second pixel based on identifying the optimal scale for the at least one second pixel.

According to another aspect of the present disclosure, an ultrasound system includes an interface, a memory, and a processor. The interface receives two-dimensional ultrasound images. The memory stores instructions and the two-dimensional ultrasound images. The processor executes the instructions. When executed by the processor, the instructions cause the ultrasound system to: for each of a plurality of pixels of each two-dimensional ultrasound image, identify an optimal scale for applying surface shading based on a size of at least one vessel in the two-dimensional ultrasound image; for each pixel of the plurality of pixels of each two-dimensional ultrasound image, selecting a corresponding filter from a filter bank based on identifying the optimal scale for the pixel and obtaining a luminance pixel corresponding to the pixel from the corresponding filter; and applying surface shading to each pixel of the plurality of pixels using luminance pixels from filters from the filter bank based on identifying the optimal scale for the pixel.

These and other aspects of the invention will be apparent from and elucidated with reference to the embodiments described hereinafter.

BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.

Fig. 1 illustrates a system for scale-adaptive ultrasound surface shading, in accordance with a representative embodiment.
Fig. 2 illustrates another system for scale-adaptive ultrasound surface shading, in accordance with a representative embodiment.
Fig. 3A illustrates a method for scale-adaptive ultrasound surface shading, in accordance with a representative embodiment.
Fig. 3B illustrates another method for scale-adaptive ultrasound surface shading, in accordance with a representative embodiment.
Fig. 4 illustrates the effects of varied filter scaling in scale-adaptive ultrasound surface shading, in accordance with a representative embodiment.
Fig. 5 illustrates a hybrid flow for scale-adaptive ultrasound surface shading, in accordance with a representative embodiment.
Fig. 6 illustrates scale estimation in scale-adaptive ultrasound surface shading, in accordance with a representative embodiment.
Fig. 7 illustrates comparative single-scale and multi-scale approaches in scale-adaptive ultrasound surface shading, in accordance with a representative embodiment.
Fig. 8 illustrates comparative effects of final shading obtained using single-scale and multi-scale approaches in scale-adaptive ultrasound surface shading, in accordance with a representative embodiment.
Fig. 9 illustrates a computer system, on which a method for scale-adaptive ultrasound surface shading is implemented, in accordance with another representative embodiment.

DETAILED DESCRIPTION OF EMBODIMENTS

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials, and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a,' 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, the technique of creating pseudo-3D appearances by adding virtual lighting effects to ultrasound images to improve visualization of blood vessels may be enhanced by applying surface shading calculations at multiple scales and choosing optimal scales on a pixel-by-pixel basis, such as to account for multiple vessel sizes present in a single image.

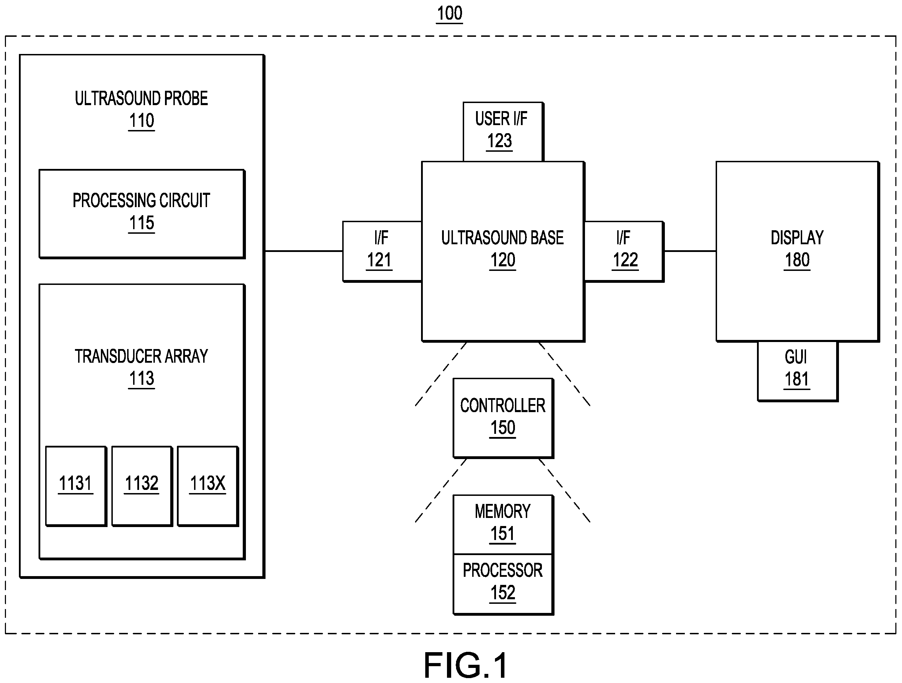

Fig. 1 illustrates a system 100 for scale-adaptive ultrasound surface shading, in accordance with a representative embodiment.

The system 100 in Fig. 1 is a system for scale-adaptive ultrasound surface shading and includes components that may be provided together or that may be distributed. The system includes an ultrasound probe 110, an ultrasound base 120 and a display 180.

The ultrasound probe 110 includes at least a transducer array 113 and a processing circuit 115. The transducer array 113 includes an array of individual transducer elements including a first transducer element 1131, a second transducer element 1132 through an Xth transducer element 113X. The transducer array 113 may include more than three transducer elements, such as dozens, hundreds or even thousands. The processing circuit 115 may include a combination of a memory that stores instructions and a processor that executes the instructions to, for example, process ultrasound imagery received via the transducer array 113. The processing circuit 115 may also or alternatively include circuit elements of an application-specific integrated circuit (ASIC).

The ultrasound base 120 includes a first interface 121, a second interface 122, a third interface 123, and a controller 150. The third interface 123 is a user interface and may be used by users to interact with the ultrasound base 120 such as to enter instructions and receive output. In Fig. 1, the controller 150 includes a memory 151 that stores instructions and a processor 152 that executes the instructions. The controller 150 in Fig. 1 also may include the third interface 123 and may also be considered to include the display 180 as a user interface in some embodiments.

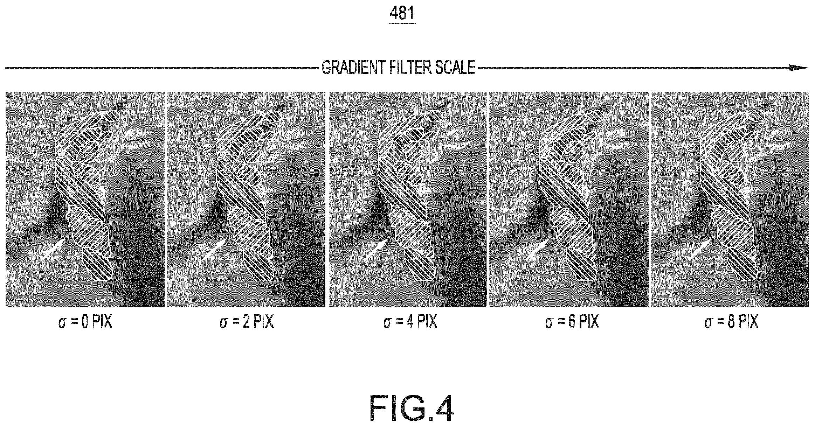

The controller 150 includes at least a memory 151 that stores instructions and a processor 152 that executes the instructions. A computer that can be used to implement the controller 150 is depicted in Fig. 4, though a controller 150 may include more or fewer elements than depicted in Fig. 1 or Fig. 4. In some embodiments, multiple different elements of the system 100 in Fig. 1 may include a controller such as the controller 150.

The display 180 may be local to the controller 150 or may be remotely connected to the controller 150. The display 180 includes a graphical user interface 181 (GUI) that may be used to display various types of images and data described herein. The display 180 may be connected to the controller 150 via a local wired interface such as an Ethernet cable or via a local wireless interface such as a Wi-Fi connection. The display 180 may be interfaced with other user input devices by which users can input instructions, including mouses, keyboards, thumbwheels and so on. The display 180 may be a monitor such as a computer monitor, a display on a mobile device, an augmented reality display, a television, an electronic whiteboard, or another screen configured to display electronic imagery. The display 180 may also include one or more input interface(s) such as those noted above that may connect to other elements or components, as well as an interactive touch screen configured to display prompts to users and collect touch input from users.

The controller 150 may perform some of the operations described herein directly and may implement other operations described herein indirectly. For example, the controller 150 may indirectly control operations such as by generating and transmitting content to be displayed on the display 180. The controller 150 may directly control other operations such as logical operations performed by the processor 152 executing instructions from the memory 151 based on input received from electronic elements and/or users via the interfaces. Accordingly, the processes implemented by the controller 150 when the processor 152 executes instructions from the memory 151 may include steps not directly performed by the controller 150.

When executed by the processor 152, instructions stored in the memory 151 may cause the system 100 to: for each of a plurality of pixels of a two-dimensional ultrasound image, identify an optimal scale for applying surface shading based on a size of at least one vessel in the two-dimensional ultrasound image. The plurality of pixels may comprise all pixels in the two-dimensional ultrasound image or may comprise a subset of all pixels in the two-dimensional ultrasound image, such as every other pixel in rows and/or columns of pixels in the entire two-dimensional ultrasound image, and/or pixels in a portion of the two-dimensional ultrasound image that does not include the entire two-dimensional ultrasound image. The instructions also cause the system 100 to apply surface shading to at least one first pixel based on identifying the optimal scale for the at least one first pixel; and apply surface shading to at least one second pixel based on identifying the optimal scale for the at least one second pixel. In some embodiments, the optimal scale for the at least one first pixel and the at least one second pixel is the same, and thus is a single universal scale such as an average optimal scale calculated on the entire image by taking into account all image pixels of the image. In other embodiments, the surface shading applied to the at least one first pixel is at a first scale based on identifying the first scale as an optimal scale for the at least one first pixel; and the surface shading applied to the at least one second pixel is at a second scale based on identifying the second scale as an optimal scale for the at least one second pixel.

In Fig. 1, the controller 150 may include two separate modules. The two separate modules may be separable in terms of programs stored in the memory 151, and/or in terms of different instances of the processor 152 or an application-specific application circuit (ASIC) used to implement the modules. The first module may automatically compute the scale of the vessel(s) present in an RGB image to present an optimal lighting effect. Parameter optimization is automated to render the controller 150 more "plunkable" with less manual tuning needed, which accelerates workflow. The second module may locally process the scale of a luminance image to generate a luminance map automatically adapted to different regions of the two-dimensional ultrasound image where anatomical structures of different sizes can be present. The second module improves the visual aspect of the vessels when multiple vessel sizes are present while offering a better differentiation of vessels.

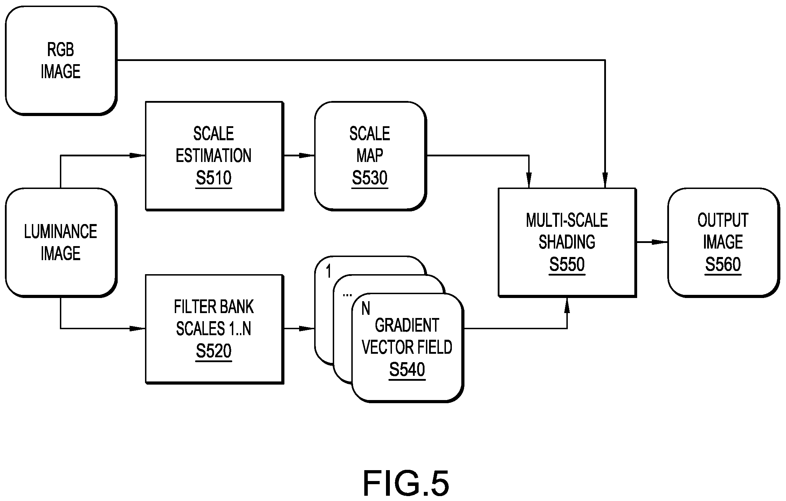

The system 100 combines three relevant elements which may be implemented by modules which are further detailed in relation to Fig. 5. The modules may be implemented as or by hardware elements such as application-specific integrated circuits and/or as or by software elements executed by one or more processor. The first element may comprise a scale estimation module, which takes as input one or more images and produces a scale map. The scale map is a two-dimensional image providing a per-pixel estimate of the size of structures present in the vicinity of that pixel. The second element is a multi-scale filtering approach that processes the luminance image to generate a luminance map which is used to produce a smooth gradient vector field that is adapted to the local vessel scale. The filtering approach may be based on a low-pass first-order derivative filter bank that convolves an input image with N > 1 filters of increasing scale and computes a set of N finite-difference gradient vector fields, one for each scale. The third element may comprise a multi-scale shading module that applies shading calculations according to a surface reflection model (e.g., Blinn-Phong), using a different gradient vector for each pixel to compute the surface normal, according to the (input) scale map value at that pixel.

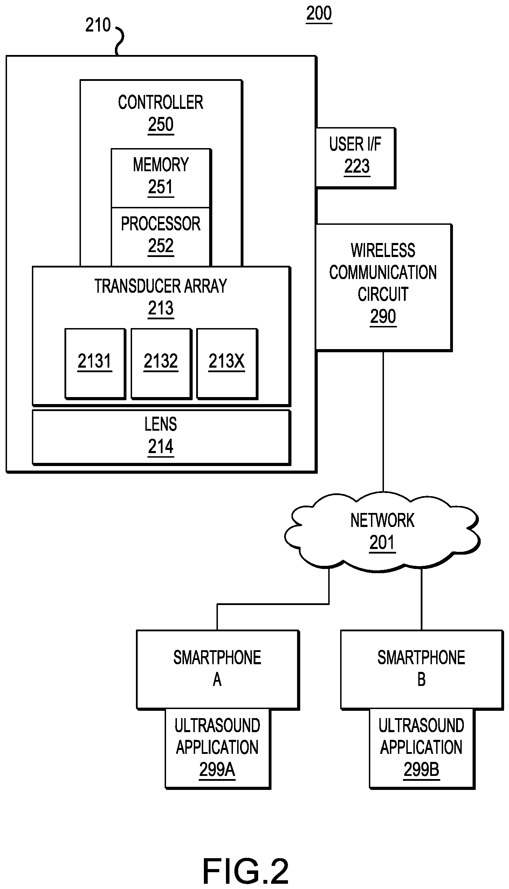

Fig. 2 illustrates another system for scale-adaptive ultrasound surface shading, in accordance with a representative embodiment.

The system 200 in Fig. 2 includes an ultrasound system 210. Additionally, while smartphone A and smartphone B are not necessarily considered part of the system 200 in Fig. 2, the ultrasound application 299A on smartphone A and the ultrasound application 299B on smartphone B may be considered part of the system 200 in Fig. 2. Although not shown, the arrangement in Fig. 2 may also include a meter to measure arterial pressure.

The ultrasound system 210 includes a transducer array 213, a lens 214, a controller 250, a user interface 223 and a wireless communication circuit 290. In Fig. 2, the controller 250 includes a memory 251 that stores instructions and a processor 252 that executes the instructions. The controller 250 may also be considered to include the user interface 223 in some embodiments. In some embodiments, smartphone A and smartphone B may be considered part of the system 200, such as when issued by the same entity that owns or otherwise controls the ultrasound system 210. Smartphone A and smartphone B communicate with the ultrasound system 210 via network 201 and the wireless communication circuit 290. The network 201 may comprise one or more of a wireless local area network (LAN) such as a Wi-Fi network and/or a wired or wireless wide area network (WAN) such as a cellular network and/or the Internet. The ultrasound system 210 may comprise an ultrasound probe such as a portable ultrasound probe that coordinates ultrasound exams with the ultrasound application 299A and/or the ultrasound application 299B.

When executed by the processor 252, instructions stored in the memory 251 may cause the system 200 to: for each of a plurality of pixels of each two-dimensional ultrasound image, identify an optimal scale for applying surface shading based on a size of at least one vessel in the two-dimensional ultrasound image. The plurality of pixels may comprise all pixels in the two-dimensional ultrasound image or may comprise a subset of all pixels in the two-dimensional ultrasound image, such as every other pixel, and/or pixels in a portion of the two-dimensional ultrasound image that does not include the entire two-dimensional ultrasound image. The instructions also cause the system 200 to apply surface shading to at least one first pixel based on identifying the optimal scale for the at least one first pixel; and apply surface shading to at least one second pixel based on identifying the optimal scale for the at least one second pixel. In some embodiments, instead of being implemented by the controller 250, some or all of the functionality attributed to the controller 150 in Fig. 1 may be executed by smartphone A or smartphone B in Fig. 2. Additionally, in some embodiments, the optimal scale for the at least one first pixel and the at least one second pixel is the same, and thus is a single universal scale such as an average optimal scale calculated on the entire image by taking into account all image pixels for the image. In other embodiments, the surface shading applied to the at least one first pixel is at a first scale based on identifying the first scale as an optimal scale for the at least one first pixel; and the surface shading applied to the at least one second pixel is at a second scale based on identifying the second scale as an optimal scale for the at least one second pixel.

In Fig. 2, the controller 250 may include two separate modules. The two separate modules may be separable in terms of programs stored in the memory 251, and/or in terms of a different processor or application-specific application circuit (ASIC) used to implement the modules. The first module may automatically compute the scale of the vessel(s) present in an RGB image to present an optimal lighting effect. Parameter optimization is automated to render the controller 150 more "plunkable" with less manual tuning needed, which accelerates workflow. The second module may locally process the scale of the luminance image to generate a luminance map automatically adapted to different regions of the two-dimensional ultrasound image where anatomical structures of different sizes can be present. The second module improves the visual aspect of the vessels when multiple vessel sizes are present while offering a better differentiation of vessels. In some embodiments, the first module and second module may be implemented in separate elements, such as if the first module is implemented in the controller 250 and instances of the second module are implemented in smartphone A and smartphone B. In some embodiments, the first module and second module may be implemented in smartphone A and smartphone B rather than in the controller 250.

The arrangement in Fig. 2 combines three relevant elements which may be implemented by modules which are further detailed in relation to Fig. 5. The modules may be implemented as or by hardware elements such as application-specific integrated circuits and/or as or by software elements executed by one or more processor. The first element may comprise a scale estimation module, which takes as input one or more images and produces a scale map. The scale map is a two-dimensional image providing a per-pixel estimate of the size of structures present in the vicinity of that pixel. The second element is a multi-scale filtering approach that processes the luminance image to generate a luminance map which is used to produce a smooth gradient vector field that is adapted to the local vessel scale. The filtering approach may be based on a low-pass first-order derivative filter bank that convolves an input image with N > 1 filters of increasing scale and computes a set of N finite-difference gradient vector fields, one for each scale. The third element may comprise a multi-scale shading module that applies shading calculations according to a surface reflection model (e.g., Blinn-Phong), using a different gradient vector for each pixel to compute the surface normal, according to the input scale map value at that pixel.

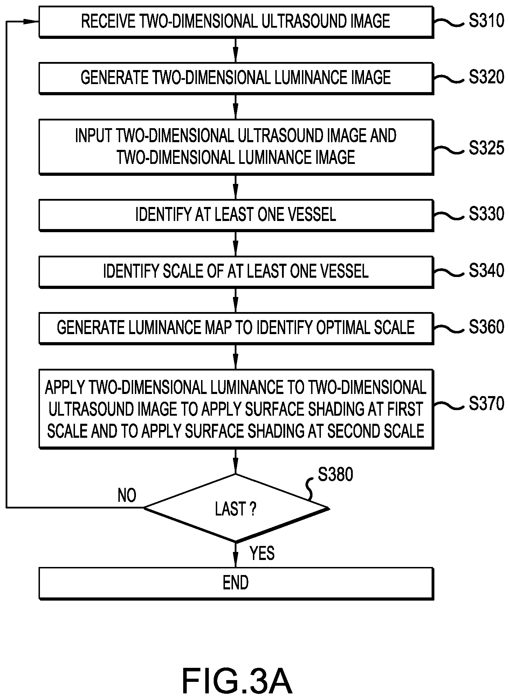

Fig. 3A illustrates a method for scale-adaptive ultrasound surface shading, in accordance with a representative embodiment.

The method of Fig. 3A may be performed by the system 100 including the controller 150 in Fig. 1, or by one or more of the controller 250 and/or smartphone A and/or smartphone B in Fig. 2.

The method of Fig. 3A starts at S310 with receiving a two-dimensional ultrasound image. S310 may include receiving a single two-dimensional ultrasound image, or more than one two-dimensional ultrasound image and then selecting one of the two-dimensional ultrasound images such as on a quality control basis. The two-dimensional ultrasound image may comprise a two-dimensional red/green/blue (RGB) ultrasound image, such as a color-coded Doppler velocity map.

At S320, a two-dimensional luminance image is received or generated. The two-dimensional luminance image may comprise the power component of a Doppler signal which is the basis of a Doppler velocity map when the two-dimensional ultrasound image comprises a color-coded Doppler velocity map.

At S325, the two-dimensional ultrasound image and the two-dimensional luminance image are input. For example, the two-dimensional ultrasound image and the two-dimensional luminance image may be input for processing by a hardware module in, and/or by a software module executed by the controller 150, the controller 250, or smartphone A or smartphone B as described herein.

At S330, at least one vessel is identified. More than one vessel may be identified, and each of the multiple such vessels may be of a different size. The vessel(s) may be identified using an image analysis program that is trained to identify vessels and characteristics of such vessels such as the size of each of the vessel(s). For example, the at least one vessel may include a first vessel of a first size and a second vessel of a second size.

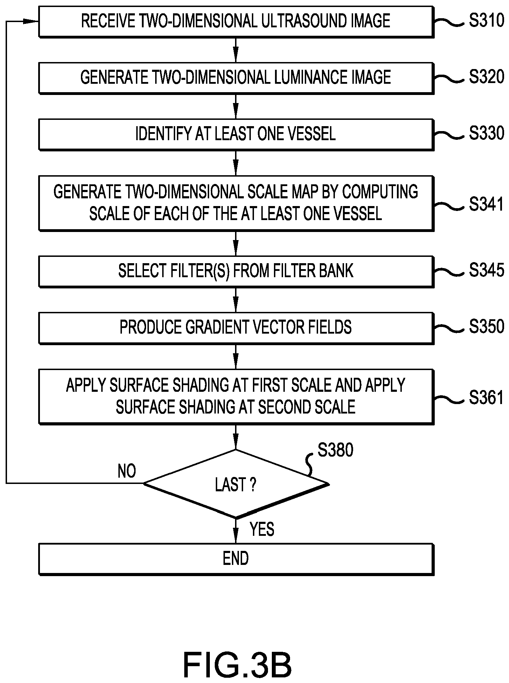

At S340, a scale of each of the at least one vessel is computed. For each of a plurality of pixels in the two-dimensional ultrasound image, an optimal scale may be identified. The optimal scale for each pixel is for applying surface shading based on a size of the at least one vessel in the two-dimensional ultrasound image. The method of Fig. 3 may include automatically computing a scale of each of at least one vessel in each two-dimensional ultrasound image to produce an optimal lighting effect for the two-dimensional ultrasound image. For example, estimated vessel sizes may range in values between radiuses of 1 pixel to 32 pixels, and scales may be computed for each vessel based on this range of values for pixels. As an example, a scale for a first vessel may be computed as 1 pixel, and a scale for a second vessel may be computed as 3 pixels, with a prefilter range (sigma) between .25 pixels and 8 pixels.

For example, when the vessels identified at S330 include the first vessel and the second vessel, first pixels may be for the first vessel and the first scale may be identified based on the first size of the first vessel. Second pixels may be for the second vessel and the second scale may be identified based on the second size of the second vessel. One or more scale(s) for one or more pixel(s) may be identified based on the first size of the first vessel and the second size of the second vessel, along with other factors such as proximity to a nearest edge of the first vessel and/or the second vessel.

At S360, a luminance map is generated to identify an optimal scale. The method of Fig. 3 may include generating, for each two-dimensional ultrasound image, a luminance map adapted for sizes of each of the at least one vessel to identify the optimal scale for applying surface shading to the two-dimensional ultrasound image. The luminance map may map a different optimal scale for each pixel in the two-dimensional ultrasound image, or for subsets of pixels in the two-dimensional ultrasound image such as every other pixel in a row and/or column, and/or pixels in a portion of the two-dimensional ultrasound image that is less than all of the two-dimensional ultrasound image. As noted, estimated vessel sizes in radius may range in value from 1 pixel to 32 pixels, and a prefilter scale (sigma) may range between .25 pixels and 8 pixels. A different optimal scale may be mapped in the luminance map for each pixel, or for subsets of pixels, based on these ranges.

At S370, two-dimensional luminance is applied to the two-dimensional ultrasound image by applying the luminance map from S360 to apply surface shading at a first scale and to apply surface shading at a second scale. As explained herein, the luminance map is a derivative of the original two-dimensional luminance image and may comprise a gradient vector field that is customized for each pixel so that surface shading may be applied to the at least one first pixel and to the at least one second pixel. The surface shading is applied to render a three-dimensional appearance to the two-dimensional ultrasound image.

The application of two-dimensional luminance at S370 may include applying surface shading to at least one first pixel at a first scale based on identifying the first scale as an optimal scale for the at least one first pixel. The application of two-dimensional luminance at S370 may also include applying surface shading to at least one second pixel at a second scale based on identifying the second scale as an optimal scale for the at least one second pixel. More than two scales may be used at S370, as each of a plurality of pixels may be applied at different scales.

At S380, a determination is made as to whether the two-dimensional ultrasound image(s) received at S310 are the last two-dimensional ultrasound image(s) to be received. If the two-dimensional ultrasound image(s) received at S310 are the last (S380 = Yes), the method ends.

If the two-dimensional ultrasound image(s) received at S310 are not the last (S380 = No), the method returns to S310 to receive one or more additional two-dimensional ultrasound image(s).

Fig. 3B illustrates another method for scale-adaptive ultrasound surface shading, in accordance with a representative embodiment.

The method of Fig. 3B may be performed by the system 100 including the controller 150 in Fig. 1, or by one or more of the controller 250 and/or smartphone A and/or smartphone B in Fig. 2.

The method of Fig. 3B again starts at S310 with receiving a two-dimensional ultrasound image. S310 may include receiving a single two-dimensional ultrasound image, or more than one two-dimensional ultrasound image and then selecting one of the two-dimensional ultrasound images such as on a quality control basis.

At S320, a two-dimensional luminance image is received or generated.

At S330, at least one vessel is identified.

At S341, a two-dimensional scale map is generated by computing a scale of each of the at least one vessel. A two-dimensional scale map may be generated for each two-dimensional ultrasound image. The scale map may reflect the first scale for the at least one first pixel and the second scale for the at least one second pixel based on sizes of vessels present in the two-dimensional ultrasound image near the at least one first pixel and near the at least one second pixel.

At S345, one or more filter(s) is or are selected from a filter bank. The filter(s) may include a first filter selected from the filter bank based on the first scale for each at least one first pixel, and a second filter for each at least one second pixel.

At S350, one or more gradient vector field(s) is or are produced. A gradient vector field is to be understood as an image comprising a two-dimensional array of three-dimensional vectors. The gradient vector field(s) may be adapted to the sizes of vessels in the two-dimensional ultrasound image near the at least one first pixel and near the at least one second pixel. The gradient vector field(s) may be produced using the first filter and the second filter selected from the filter bank at S345.

At S361, surface shading is applied at a first scale and surface shading is applied at a second scale. The surface shading may be applied to each two-dimensional ultrasound image using a different gradient vector for each of the at least one first pixel and the at least one second pixel.

At S380, a determination is made as to whether the two-dimensional ultrasound image(s) received at S310 are the last two-dimensional ultrasound image(s) to be received. If the two-dimensional ultrasound image(s) received at S310 are the last (S380 = Yes), the method ends.

If the two-dimensional ultrasound image(s) received at S310 are not the last (S380 = No), the method returns to S310 to receive one or more additional two-dimensional ultrasound image(s).

Fig. 4 illustrates the effects of varied filter scaling in scale-adaptive ultrasound surface shading, in accordance with a representative embodiment.

Fig. 4 illustrates the varying levels of detail that result for five different gradient filter scales used for the same underlying input RGB image. As shown, the lowest scale for the input RGB image on the left shows the most detail in terms of depth for the tissue pointed to by the arrow. The level of detail decreases for each input RGB image to the right, so that the highest scale for the RGB image on the right shows the lowest detail in terms of depth for the tissue pointed to by the arrow. Using the system 100 in Fig. 1 or the arrangement in Fig. 2, scale may be optimized automatically according to the size of the vessels present in the RGB image so as to reach an optimal result in terms of visual separation. The automated scale may be applied to RGB images in which multiple vessel sizes are present.

Fig. 4 may reflect a conventional colorized ultrasound image with colors in red, blue, gray, and black. Luminance values (virtual lighting) may be adjusted for individualized pixels reflected in Fig. 4 to provide a virtualized 3-dimensional appearance. The luminance values may vary to provide estimates of different heights/depths of surfaces in the ultrasound image in Fig. 4, wherein the correspondence is generally that the brighter the pixel, the higher the surface at the pixel. The gray and black colors are for the background B-mode in the ultrasound image. The blue and red colors show anatomical structures with opposite blood flow directions relative to the transducer such as vessels and veins, and the shading reflected by varied luminance values provide highlights for the virtualized 3-dimensional impression. A different channel than the colorized ultrasound image is used to provide the highlights for the 3-dimensional effects in the colorized ultrasound image by constructing a surface. The color in the colorized ultrasound image depends on the blood flow velocity estimated from the Doppler signals, whereas the height of the surface depends on the power of the Doppler signals used to estimate the blood flow velocity.

There are at least two main shortcomings from conventional colorizing of ultrasound images. The first problem is that a single scale parameter is used to prefilter the ultrasound image before colorizing. Different scales provide completely different effects as shown in Fig. 4. Fig. 4 illustrates that the optimal scale value is directly related to the size of the vessel currently scanned. Therefore, choosing a single scale in advance should ideally not be the basis of scaling since the vessel size varies widely as a function of the anatomical view and patient under examination.

The second problem is that ultrasound images typically include multiple vessels of different sizes (for example, a large vein such as the portal vein and a small artery such as the hepatic artery). Therefore, even if the user could interactively adjust the single scale through a UI control, it would be impossible to select a single scale that is optimum for all vessels present in different parts of the same ultrasound image. Furthermore, the optimum scale is actually determined by the *local vessel size* which can vary widely from one pixel to another so, ideally, the scale should be adaptively adjusted at different parts of the image to match the local vessel size.

As explained herein, the first problem may be addressed by automatically estimating the size of a vessel in an image and then setting scale values pixel-by-pixel for the image based on the size of the vessel. The second problem may be addressed by automatically estimating the local vessel size in a pixel-by-pixel manner. The result may be scaling based on vessel size and/or multi-scaling.

An optimal scale may be produced for each pixel on a scale map based on the input ultrasound image. One scale may be produced for the entire image, and one scale value may be produced for each pixel of the image, as this may provide an optimal scale by automatic means. Alternatively, different scales may be produced such as by estimating scales automatically for each vessel by estimating the local vessel size on a pixel-by-pixel basis.

The lighting that usually comes from a single scale is now performed pixel-by-pixel. The one scale value produced for the entire image is now produced automatically instead of being set by the user. The multiscale values produced pixel-by-pixel are used to calculate the lighting. The multi-scale map is pixel-by-pixel but does not have to be for every pixel in the ultrasound image. Interpolation may be used, and any level of resolution may be used. Also, the pixel-by-pixel scale map may be of a different overall size than the ultrasound image, like for only a subset of the ultrasound image. The scale can be estimated for every other pixel for instance.

Fig. 5 illustrates a hybrid flow for scale-adaptive ultrasound surface shading, in accordance with a representative embodiment.

In Fig. 5, processing of a luminance image is performed at S510, S520, S530, S540 and S550. Processing of an RGB image is also performed at S550.

Specifically, scale estimation is applied to the luminance image at S510, and a scale map is generated based on the scale estimation at S530.

Filter bank scales #1... N are applied to the luminance image at S520 to produce #1...N gradient vector fields at S540. As noted previously, the filtering approach may be based on a low-pass first-order derivative filter bank that convolves an input image with N > 1 filters of increasing scale and computes a set of N finite-difference gradient vector fields, one for each scale. For example, a set of gradient vector fields may include seven gradient vector fields for seven different scales.

The scale map generated at S530 is applied to the RGB image in multi-scale shading at S550 using the 1...N gradient vector fields produced at S540.
At S560, the result of applying the scale map to the RGB image using the 1...N gradient vector fields at S550 is output as an output image.

In Fig. 5, the starting points are a 2-dimensional RGB image and a luminance image. The luminance image is input to scale estimation processing at S510 to produce a scale map at S530. A filter bank is applied to the luminance image at S520 to produce a gradient vector field at S540. The scale map and the gradient vector field resulting from the filter bank are combined at S550 with the RGB image to apply multi-scale shading, and the result is output as an output image. The RGB image and the luminance image are for the same underlying ultrasound image. The RGB image may first be segmented, regardless of whether a global single scaled version is intended or a pixel-by-pixel version is intended.

The three relevant elements added by the teachings relating to FIG. 5 herein include the scale estimation applied to the luminance image at S510 by a first module, the filter bank scales #1...N applied to the luminance image at S520, and the multi-scale shading at S550 by a second module. The first element may comprise a scale estimation module, which takes as input one or more images and produces a scale map. The scale map is a two-dimensional image providing a per-pixel estimate of the size of structures present in the vicinity of that pixel. The second element is a multi-scale filtering approach that processes the luminance image to generate a luminance map which is used to produce a smooth gradient vector field that is adapted to the local vessel scale. The filtering approach may be based on a low-pass first-order derivative filter bank that convolves an input image with N > 1 filters of increasing scale and computes a set of N finite-difference gradient vector fields, one for each scale. The third element may comprise a multi-scale shading module that applies shading calculations according to a surface reflection model (e.g., Blinn-Phong), using a different gradient vector for each pixel to compute the surface normal, according to the input scale map value at that pixel.

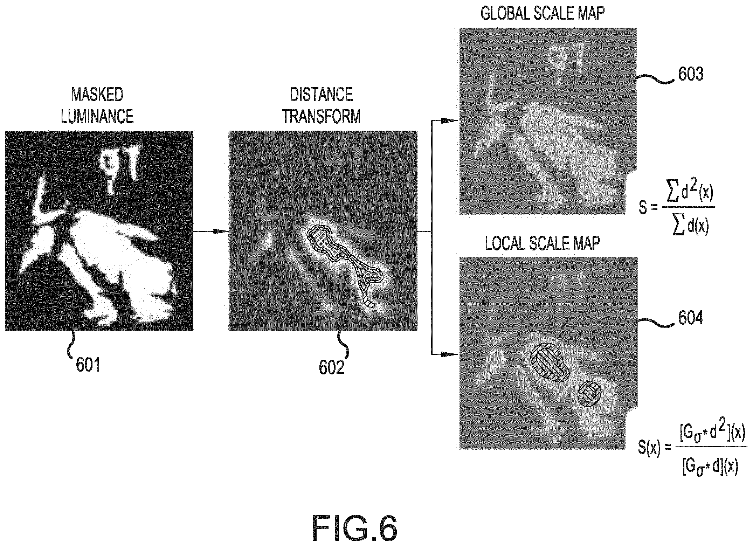

Fig. 6 illustrates scale estimation in scale-adaptive ultrasound surface shading, in accordance with a representative embodiment.

Fig. 6 shows the processing flow of a first module to estimate the scale. A luminance image is first masked by applying a threshold that depends on the noise level. Based on the binary mask, an exact Euclidean distance transform, encoding the distance of any given pixel to the closest boundary point, may be generated, using, for example, the Maurer algorithm. These first steps are common to the single-scale and multi-scale approaches.

In the single-scale approach, the automatically adapted optimal scale (S) can be estimated by approximating the radius along the centerline, as follows:

$$S = \frac{\sum d^2(x)}{\sum d(x)}$$

where d(x) is the value of the distance transform at pixel x. The higher d(x) is, the closer x is to the centerline, or medial axis of the shape. Rather than calculating a standard average distance inside the shape, which would underestimate the vessel size, the above weighted average equation approximates the average radius along the centerline by putting more weight on high distance values, which correspond to points closer to the centerline, and less weight on low distances that correspond to points closer to the boundary. The advantage of this technique is its genericity and limited complexity, as it does not require an explicit calculation of centerline curves.

The same principle may be used to estimate the scale locally, instead of globally on the entire shape. To do so, the distance transform is processed locally using a Gaussian kernel of standard deviation $\sigma$ as follows:

$$S(x) = \frac{[G_\sigma * d^2](x)}{G_\sigma d(x)}$$

In Fig. 6, masked luminance is shown at 601 and is applied to produce a distance transform at 602. The distance transform is applied to produce a global scale map at 603 and a local scale map at 604. The global scale map at 603 is based on a ratio of the sum of distance^2(x) to the sum of distance(x). The local scale map at 604 is based on a ratio of scale factors G_$\sigma$*distance^2(x) to G_o *distance(x).

In Fig. 6, for scale estimation as at S510 in Fig. 5, the luminance image may be subject to a threshold to produce the masked luminance at 601 as shown in black and white. The masked luminance at 601 reflects whether a signal is above or below the threshold, so if below the threshold the color signal is a black pixel and if above the threshold the color signal is valid and is a white pixel. The binary image for masked luminance at 601 in Fig. 6 provides a shape of the different vessels already in the ultrasound image, and this may be key to determining for each pixel, what is the size of the vessel.

Next in Fig. 6, a distance transform at 602 is generated. The distance transform at 602 is generated from the masked luminance at 601 by providing a variable value that reflects the distance of the pixel to the closest edge of the vessel. The color blue may be used to reflect a low distance from the closest edge, the color yellow may be used to reflect a relatively medium distance from the closest edge, and the color red may be used to reflect a relative high distance from the closest edge. The color red may be used for the center(s) of the large vessel since these centers have the highest distances to edges of vessels in the distance transform at 602. The global scale map at 603 may reflect application of the first summing function shown to the right to the distance transform at 602, whereas the local scale map at 604 may reflect application of the second summing function shown to the right to the distance transform at 602. The global scale map at 603 may have a binary appearance reflecting a dedicated average using a single scale value, whereas the local scale map at 604 may have more than 2 colors such as dark blue outside of the vessels, light blue for the smaller vessels and around the edges of the larger vessel, and yellow at the center(s) of the large vessel. The local scale map relates to different scales that vary for pixels based on the size of the vessel for the pixel. The local scale map at 604 may be pixel by pixel, or for subsets of pixels such as every other pixel.

The global scale map 603 may be analogized in simple terms for a perfect vessel such as a solid tube with a centerline and a uniform radius to the edges of the tube from the centerline. For each point of the centerline, an estimate may be generated for the size of the vessel, so that the scale would reflect an average of the size estimates generated for each point along the centerline. The global scale then provides an estimate of the size of the solid tube as the vessel.

Almost no vessel will have a perfect structure such as a solid tube. However, the distance transform at 602 reflects the highest values which would appear at the relative center of the vessel, so the distance transform at 602 provides a proxy for the centerline. For the equations, the distance d is used as a weight in a weight map to average the distance for the global scale map at 603. The average along the central line is taken, weighted by how likely a pixel is to be on the central line, and the resulting likelihood is proportional to the distance.

Based on the processing in Fig. 6, pixels for two anatomical structures of the same size may have similar or identical luminance values even though the two structures are of different types, such as organ and vein. On the other hand, pixels for two anatomical structures of different sizes may have substantially different luminance values even though the two structures are of the same type, such as two veins. The similar luminance values or the different luminance values may result even when the two anatomical structures are intertwined insofar as the processing distinguishes between different anatomical structures. The luminance values are assigned based on the scale reflecting the size of the anatomical structure for the pixel. The luminance value is a function of the size of the anatomical structure.

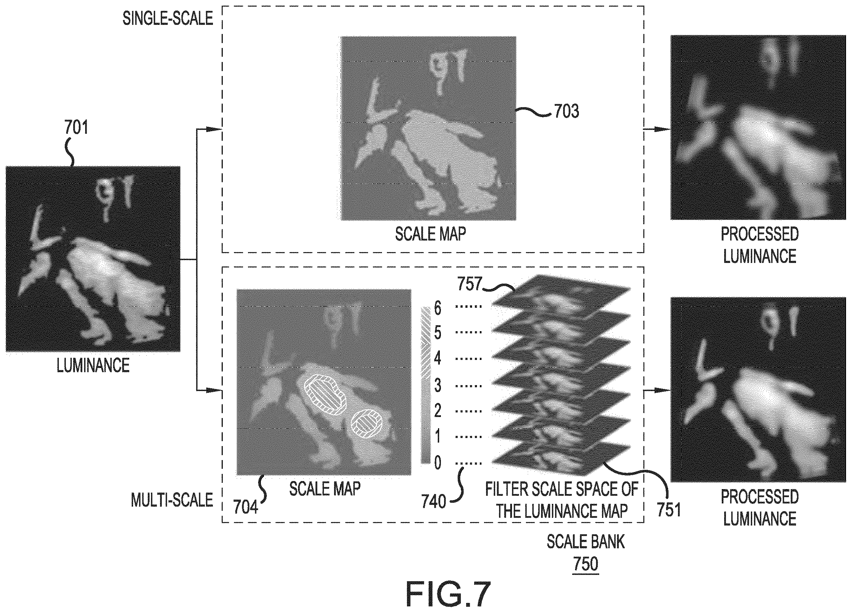

Fig. 7 illustrates comparative single-scale and multi-scale approaches in scale-adaptive ultrasound surface shading, in accordance with a representative embodiment. That is, Fig. 7 compares the single-scale and multi-scale approaches for the processing of the luminance image to generate a luminance map.

In the single-scale method, the luminance image may be processed with a low pass filter at scale $\sigma$ given by the global scale map computed by the first module as in Fig. 6.

In the multi-scale approach, the luminance image may be processed per pixel, based on the local scale value. To that purpose, a multi-scale implementation based on a Gaussian scale space may be used. A scale space is generated by applying a low pass filter at all the scales obtained in the local scale map. The scale map is then used to select the most appropriate scale for each pixel. The implementation makes no per pixel binary decisions facilitating the temporal coherence of the processing. In addition, the implementation does not use image intensity information making it independent from the image type. To reduce the computational cost, the filter bank may be computed only on a limited number of scales. The value of the processed luminance map for a pixel at a given scale $s\_(n + \varepsilon)$ is obtained by linear interpolation between the values of the pixel at the consecutive scales $s\_n$ and $s\_(n+1)$.

In Fig. 7, a luminance map is shown at 701, and is applied to produce a single-scale scale map at 703 and a multi-scale scale map at 704. The luminance map at 701 may be provided along with an RGB image for processing as described herein. A scale space 740 is shown for a filter scale bank 750 that includes seven filter scale maps from first filter scale map 751 to seventh filter scale map 757. The seven filter scales comprise a filter scale space of the luminance map and can be selectively applied on a pixel-by-pixel basis to the multi-scale scale map at 704 to produce a processed luminance map on the bottom right. The single-scale scale map at 703 is applied to the luminance map at 701 to produce a processed luminance map on the top right.

In Fig. 7, multiple scale maps may be provided in a filter scale bank 750. Seven different scale maps are shown from a first filter scale map 751 to a seventh filter scale map 757. A luminance value for a pixel depends on the size of the local anatomical structure. In Fig. 7, the filter scale maps in filter scale bank 750 are used to establish the luminance values for the processed luminance map on the bottom right in the context of doing virtual lighting.

In Fig. 7, the filter scale bank 750 is used in preprocessing for virtual lighting so as to pre-filter the luminance image before any subsequent processing.

Instead of a low pass filter such as a Gaussian filter used conventionally for an entire ultrasound image to apply a single scale, in Fig. 7 a filter bank is used to select a scale map for each pixel being processed so as to account for the size of the vessels represented by subsets of the pixels. In Fig. 7, the scale pre filter parameter is made dependent on the scale map from the filter bank so that the individual scale values do not require complex individualized processing. The scale space 740 may include a color chart such as from dark blue to light blue to green to yellow with 7 different colors corresponding to the filter scale maps in the filter scale bank 750. The different scale maps correspond to different levels of blurring in the images. The scale maps in the filter scale bank 750 are precalculated. As an example, a filter scale bank 750 may include four scale maps, eight scale maps, or sixteen scale maps, and the available resolutions may vary based on the number of scale maps to use. A system uses the same relative locations of pixels in the underlying image and the scale map to produce a result. Additionally, a system may interpolate using two scale maps. The resultant processed luminance maps are made dependent on the scale maps in the filter bank.

As a result of using the filter scale bank 750, a scale value for a pixel that does not correspond to a vessel may be zero, whereas scale values for pixels corresponding to vessels will vary depending on the size of the corresponding vessel. The scale maps in the filter scale bank 750 will be used to compute lighting for each pixel in a pixel-by-pixel setting.

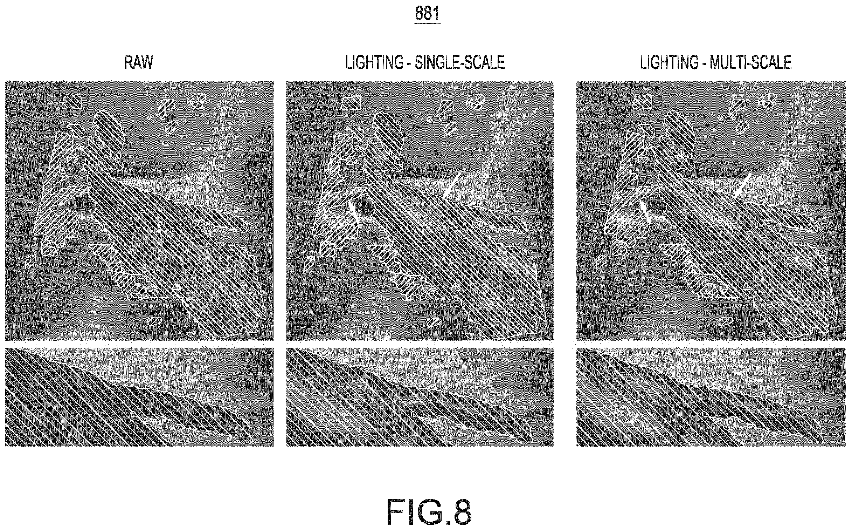

Fig. 8 illustrates comparative effects of final shading obtained using single-scale and multi-scale approaches in scale-adaptive ultrasound surface shading, in accordance with a representative embodiment.

The second module is used to implement the multi-scale shading at Fig. 8 and computes discrete gradients in the x and y directions based on the processed luminance map. The discrete gradients are then fed to a surface shading model to enlighten and provide a three-dimensional aspect to the RGB image. Fig. 8 provides a comparison of the final shading when using the single-scale and multi-scale approaches. In each of the three sets of images (left, middle, right) in Fig. 8, the hepatic artery is better differentiated from the portal vein on the image obtained with the multi-scale approach.

In Fig. 8, raw shading is shown on a user interface 881 to the left. Lighting using a single scale is shown on the user interface 881 in the middle. Lighting using multi-scale shading is shown on the user interface 881 on the right.

In Fig. 8, to apply virtual lighting to a surface, you first require a normal to the surface. The lighting calculation depends on location of the light source(s) and how the normal of the surface is oriented with respect to the light source(s). An X vector, a Y vector and a third vector normal to the surface are required. The third vector is calculated by computing the gradient of the image. The scale space is for the gradients, reflecting the difference between neighboring pixels as this provides a slope of the image as a spatial derivative of the image reflecting a slope of the surface.

In Fig. 8, the arrows point to different anatomical structures which a system differentiates in terms of using size to determine scale values. For example, one arrow may point to the larger portal vein, and the other arrow may point to a smaller hepatic artery. The virtualized lighting applied on a pixel-by-pixel basis provides an improved differentiation even when such anatomical structures are connected or close as shown by the smaller portion pointed to by the arrow on the left relative to the larger anatomical structure pointed to by the arrow on the right. Shading in colorized versions may be used to differentiate between the different vessels.

In Fig. 8, only a single scale is used for the entire image in the middle, whereas the multi-scale lighting is used for the image on the right. A user interface may clearly show an operator the details of the anatomy in Fig. 8, including different structures that are connected or otherwise near one another.

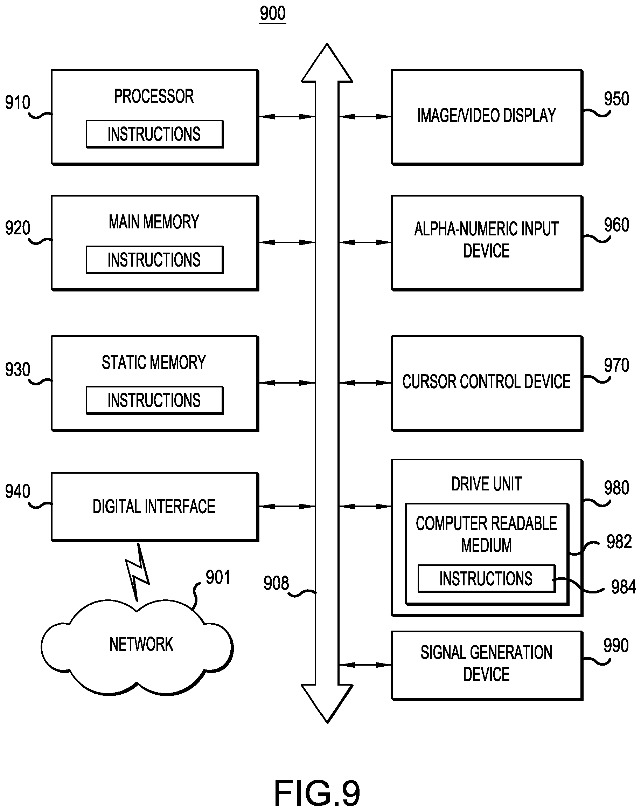

Fig. 9 illustrates a computer system, on which a method for scale-adaptive ultrasound surface shading is implemented, in accordance with another representative embodiment.

Referring to Fig. 9, the computer system 900 includes a set of software instructions that can be executed to cause the computer system 900 to perform any of the methods or computer-based functions disclosed herein. The computer system 900 may operate as a standalone device or may be connected, for example, using a network 901, to other computer systems or peripheral devices. In embodiments, a computer system 900 performs logical processing based on digital signals received via an analog-to-digital converter.

In a networked deployment, the computer system 900 operates in the capacity of a server or as a client user computer in a server-client user network environment, or as a peer computer system in a peer-to-peer (or distributed) network environment. The computer system 900 can also be implemented as or incorporated into various devices, such as a workstation that includes a controller such as the controller 150 or the controller 250, a stationary computer, a mobile computer, a personal computer (PC), a laptop computer, a tablet computer, or any other machine capable of executing a set of software instructions (sequential or otherwise) that specify actions to be taken by that machine. The computer system 900 can be incorporated as or in a device that in turn is in an integrated system that includes additional devices. In an embodiment, the computer system 900 can be implemented using electronic devices that provide voice, video, or data communication. Further, while the computer system 900 is illustrated in the singular, the term "system" shall also be taken to include any collection of systems or sub-systems that individually or jointly execute a set, or multiple sets, of software instructions to perform one or more computer functions.

As illustrated in Fig. 9, the computer system 900 includes a processor 910. The processor 910 may be considered a representative example of a processor of a controller and executes instructions to implement some or all aspects of methods and processes described herein. The processor 910 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 910 is an article of manufacture and/or a machine component. The processor 910 is configured to execute software instructions to perform functions as described in the various embodiments herein. The processor 910 may be a general-purpose processor or may be part of an application specific integrated circuit (ASIC). The processor 910 may also be a microprocessor, a microcomputer, a processor chip, a controller, a microcontroller, a digital signal processor (DSP), a state machine, or a programmable logic device. The processor 910 may also be a logical circuit, including a programmable gate array (PGA), such as a field programmable gate array (FPGA), or another type of circuit that includes discrete gate and/or transistor logic. The processor 910 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 900 further includes a main memory 920 and a static memory 930, where memories in the computer system 900 communicate with each other and the processor 910 via a bus 908. Either or both of the main memory 920 and the static memory 930 may be considered representative examples of a memory of a controller, and store instructions used to implement some, or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 920 and the static memory 930 are articles of manufacture and/or machine components. The main memory 920 and the static memory 930 are computer-readable mediums from which data and executable software instructions can be read by a computer (e.g., the processor 910). Each of the main memory 920 and the static memory 930 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 900 further includes a video display unit 950, such as a liquid crystal display (LCD), an organic light emitting diode (OLED), a flat panel display, a solid-state display, or a cathode ray tube (CRT), for example. Additionally, the computer system 900 includes an input device 960, such as a keyboard/virtual keyboard or touch-sensitive input screen or speech input with speech recognition, and a cursor control device 970, such as a mouse or touch-sensitive input screen or pad. The computer system 900 also optionally includes a disk drive unit 980, a signal generation device 990, such as a speaker or remote control, and/or a network interface device 940.

In an embodiment, as depicted in Fig. 9, the disk drive unit 980 includes a computer-readable medium 982 in which one or more sets of software instructions 984 (software) are embedded. The sets of software instructions 984 are read from the computer-readable medium 982 to be executed by the processor 910. Further, the software instructions 984, when executed by the processor 910, perform one or more steps of the methods and processes as described herein. In an embodiment, the software instructions 984 reside all or in part within the main memory 920, the static memory 930 and/or the processor 910 during execution by the computer system 900. Further, the computer-readable medium 982 may include the software instructions 984 or receive and execute the software instructions 984 responsive to a propagated signal, so that a device connected to a network 901 communicates voice, video, or data over the network 901. The software instructions 984 may be transmitted or received over the network 901 via the network interface device 940.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, scale-adaptive ultrasound surface shading enables improved visualization of blood vessels in the technique of adding virtual lighting effects to ultrasound Doppler images by creating a pseudo-3D appearance by applying surface shading calculations at multiple scales and choosing optimal scales on a pixel-by-pixel basis, such as to account for multiple vessel sizes present in a single image.

The teachings herein may be provided as an improvement to flow viewer, and/or on systems such as EPIQ systems or Affiniti systems, targeting obstetrical, vascular, and musculoskeletal applications. The teachings may be applicable to all flow imaging modes in flow viewer, so including color Doppler, power Doppler (also known as CPA), directional power Doppler (also known as dCPA), microflow Imaging (also known as MFI), and high-definition MFI (also known as MFI-HD), both during real-time imaging as well as in post-processing.

Although scale-adaptive ultrasound surface shading has been described with reference to several exemplary embodiments, it is understood that the words that have been used are words of description and illustration, rather than words of limitation. Changes may be made within the purview of the appended claims, as presently stated, and as amended, without departing from the scope of scale-adaptive ultrasound surface shading in its aspects. Although scale-adaptive ultrasound surface shading has been described with reference to particular means, materials and embodiments, scale-adaptive ultrasound surface shading is not intended to be limited to the particulars disclosed; rather scale-adaptive ultrasound surface shading extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the claims. Thus, to the maximum extent allowed by law, the scope of the present disclosure is to be determined by the broadest permissible interpretation of the following claims and their equivalents and shall not be restricted or limited by the foregoing detailed description.

**Claims**

1. An ultrasound system (210), comprising:

an interface that receives a two-dimensional ultrasound image; and
a processor (152) configured to:

for each of a plurality of pixels of the two-dimensional ultrasound image, identify an optimal scale (S340/S341) for applying surface shading based on a size of at least one vessel in the two-dimensional ultrasound image;
apply surface shading (S361/S370) to at least one first pixel based on identifying the optimal scale for the at least one first pixel; and
apply surface shading (S361/S370) to at least one second pixel based on identifying the optimal scale for the at least one second pixel.

2. The ultrasound system (210) of claim 1, wherein the processor (152) is further configured to:

apply surface shading to the at least one first pixel at a first scale based on identifying the first scale as an optimal scale for the at least one first pixel; and
apply surface shading to the at least one second pixel at a second scale based on identifying the second scale as an optimal scale for the at least one second pixel,
wherein the at least one vessel includes a first vessel of a first size and a second vessel of a second size,
wherein the at least one first pixel comprises a pixel of the first vessel, and the first scale is identified based on the first size of the first vessel, and
wherein the at least one second pixel comprises a pixel of the second vessel, and the second scale is identified based on the second size of the second vessel.

3. The ultrasound system (210) of claim 1, wherein the processor (152) is further configured to:

receive a two-dimensional luminance image for the two-dimensional ultrasound image;
generate a luminance map based on the luminance image; and
apply the two-dimensional luminance map to the two-dimensional ultrasound image to apply surface shading to the at least one first pixel and to the at least one second pixel, wherein the surface shading is applied to render a three-dimensional appearance to the two-dimensional ultrasound image.

4. The ultrasound system (210) of claim 1, wherein the two-dimensional ultrasound image comprises a red/green/blue, RGB, image.

5. The ultrasound system (210) of claim 1, wherein the processor (152) is further configured to:

automatically compute a scale of each of at least one vessel in the two-dimensional ultrasound image to produce an optimal lighting effect for the two-dimensional ultrasound image; and
generate a luminance map adapted for sizes of each of the at least one vessel to identify the optimal scale for applying surface shading to the two-dimensional ultrasound image.

6. The ultrasound system (210) of claim 2, wherein processor (152) is further configured to:

generate, for the two-dimensional ultrasound image, a two-dimensional scale map which reflects the first scale for the at least one first pixel and the second scale for the at least one second pixel based on sizes of vessels present in the two-dimensional ultrasound image near the at least one first pixel and near the at least one second pixel;
produce a gradient vector field adapted to the sizes of vessels present in the two-dimensional ultrasound image near the at least one first pixel and near the at least one second pixel, and
apply shading to the two-dimensional ultrasound image using a different gradient vector from the gradient vector field for each of the at least one first pixel and the at least one second pixel.

7. The ultrasound system (210) of claim 6, wherein the gradient vector field is produced using a first filter selected from a filter bank based on the first scale for each at least one first pixel and using a second filter selected from the filter bank based on the second scale for each at least one second pixel.

8. The ultrasound system (210) of any one of claims 1 to 7 further comprising:
an interface to display the received two-dimensional ultrasound image and/or to display an ultrasound imaged processed according to any one of claims 1 to 7.

9. A method for applying virtual lighting to ultrasound images, the method comprising:

receiving a two-dimensional ultrasound image; for each of a plurality of pixels of each two-dimensional ultrasound image, identifying (S340/S341) an optimal scale for applying surface shading based on a size of at least one vessel in the two-dimensional ultrasound image; applying (S361/S370) surface shading to at least one first pixel based on identifying the optimal scale for the at least one first pixel; and applying (S361/S370) surface shading to at least one second pixel based on identifying the optimal scale for the at least one second pixel.

10. The method of claim 9, further comprising:

applying surface shading to the at least one first pixel at a first scale based on identifying the first scale as an optimal scale for the at least one first pixel; and applying surface shading to the at least one second pixel at a second scale based on identifying the second scale as an optimal scale for the at least one second pixel, wherein the at least one vessel includes a first vessel of a first size and a second vessel of a second size, wherein the first pixels are for the first vessel, and the first scale is identified based on the first size of the first vessel, and wherein the second pixels are for the second vessel, and the second scale is identified based on the second size of the second vessel.

11. The method of claim 10, further comprising:

receiving a two-dimensional luminance image for the two-dimensional ultrasound image; generating a luminance map based on the luminance image; and applying the two-dimensional luminance map to the two-dimensional ultrasound image to apply surface shading to the at least one first pixel and to the at least one second pixel, wherein the surface shading is applied to render a three-dimensional appearance to the two-dimensional ultrasound image.

12. The method of claim 9, further comprising:

automatically computing a scale of each of at least one vessel in the two-dimensional ultrasound image to produce an optimal lighting effect for the two-dimensional ultrasound image; and generating, for the two-dimensional ultrasound image, a luminance map adapted for sizes of each of the at least one vessel to identify the optimal scale for applying surface shading to the two-dimensional ultrasound image.

13. The method of claim 9, further comprising:

generating, for the two-dimensional ultrasound image, a two-dimensional scale map which reflects the first scale for the at least one first pixel and the second scale for the at least one second pixel based on sizes of vessels present in the two-dimensional ultrasound image near the at least one first pixel and near the at least one second pixel; producing a gradient vector field adapted to the sizes of vessels present in the two-dimensional ultrasound image near the at least one first pixel and near the at least one second pixel, and applying shading to the two-dimensional ultrasound image using a different gradient vector from the gradient vector field for each of the at least one first pixel and the at least one second pixel.

14. The method of claim 13, wherein the gradient vector field is produced using a first filter selected from a filter bank based on the first scale for each at least one first pixel and using a second filter selected from the filter bank based on the second scale for each at least one second pixel.

15. A computer program product comprising instructions which when executed by a processor cause the processor to execute a method according to any one of claims 9 to 14.

FIG.1

EP 4 599 773 A1

FIG.2

RECEIVE TWO-DIMENSIONAL ULTRASOUND IMAGE — S310

GENERATE TWO-DIMENSIONAL LUMINANCE IMAGE — S320

INPUT TWO-DIMENSIONAL ULTRASOUND IMAGE AND
TWO-DIMENSIONAL LUMINANCE IMAGE — S325

IDENTIFY AT LEAST ONE VESSEL — S330

IDENTIFY SCALE OF AT LEAST ONE VESSEL — S340

GENERATE LUMINANCE MAP TO IDENTIFY OPTIMAL SCALE — S360

APPLY TWO-DIMENSIONAL LUMINANCE TO TWO-DIMENSIONAL
ULTRASOUND IMAGE TO APPLY SURFACE SHADING AT FIRST
SCALE AND TO APPLY SURFACE SHADING AT SECOND SCALE — S370

NO ←— LAST ? — S380

YES

END

# FIG.3A

RECEIVE TWO-DIMENSIONAL ULTRASOUND IMAGE — S310

GENERATE TWO-DIMENSIONAL LUMINANCE IMAGE — S320

IDENTIFY AT LEAST ONE VESSEL — S330

GENERATE TWO-DIMENSIONAL SCALE MAP BY COMPUTING SCALE OF EACH OF THE AT LEAST ONE VESSEL — S341

SELECT FILTER(S) FROM FILTER BANK — S345

PRODUCE GRADIENT VECTOR FIELDS — S350

APPLY SURFACE SHADING AT FIRST SCALE AND APPLY SURFACE SHADING AT SECOND SCALE — S361

S380

NO — LAST ? — YES

END

# FIG.3B

481

GRADIENT FILTER SCALE

σ = 0 PIX       σ = 2 PIX       σ = 4 PIX       σ = 6 PIX       σ = 8 PIX

FIG.4

EP 4 599 773 A1

RGB IMAGE

LUMINANCE IMAGE

SCALE ESTIMATION S510

FILTER BANK SCALES 1..N S520

SCALE MAP S530

GRADIENT VECTOR FIELD S540

MULTI-SCALE SHADING S550

OUTPUT IMAGE S560

FIG.5

FIG.6

SINGLE-SCALE

701

LUMINANCE

SCALE MAP

703

PROCESSED LUMINANCE

MULTI-SCALE

704

SCALE MAP

6 5 4 3 2 1 0

740

757

FILTER SCALE SPACE OF THE LUMINANCE MAP

751

SCALE BANK 750

PROCESSED LUMINANCE

FIG.7

881

RAW

LIGHTING - SINGLE-SCALE

LIGHTING - MULTI-SCALE

FIG.8

<u>900</u>

FIG.9

**Europäisches Patentamt**
**European Patent Office**
**Office européen des brevets**

# EUROPEAN SEARCH REPORT

**Application Number**

EP 24 30 5205

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/322628 A1 (SCHROECKER GERALD [AT] ET AL) 8 November 2018 (2018-11-08) * paragraphs [0003] - [0007], [0015] - [0026]; claims; figures * | 1-15 | INV. A61B8/08 A61B8/00 |
| A | US 2023/316642 A1 (MORY BENOIT JEAN-DOMINIQUE BERTRAND MAURICE [US] ET AL) 5 October 2023 (2023-10-05) * paragraph [0084]; claims; figures * | 1-15 | |
| A | US 2022/005258 A1 (MORY BENOIT JEAN-DOMINIQUE BERTRAND MAURICE [US] ET AL) 6 January 2022 (2022-01-06) * paragraph [0006]; claims; figures * | 1-15 | |
| A | US 2023/073704 A1 (TREMBLAY-DARVEAU CHARLES [US] ET AL) 9 March 2023 (2023-03-09) * paragraphs [0029], [0030]; claims; figures * | 1-15 | |

**TECHNICAL FIELDS SEARCHED (IPC)**

A61B

The present search report has been drawn up for all claims

| Place of search | Date of completion of the search | Examiner |
|---|---|---|
| Munich | 3 July 2024 | Mundakapadam, S |

EPO FORM 1503 03.82 (P04C01)

## ANNEX TO THE EUROPEAN SEARCH REPORT
## ON EUROPEAN PATENT APPLICATION NO.

EP 24 30 5205

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2024

| Patent document cited in search report | Publication date | Patent family member(s) | | Publication date |
|---|---|---|---|---|
| US 2018322628 A1 | 08-11-2018 | CN | 108805946 A | 13-11-2018 |
| | | JP | 7077118 B2 | 30-05-2022 |
| | | JP | 2018187371 A | 29-11-2018 |
| | | KR | 20180122944 A | 14-11-2018 |
| | | US | 2018322628 A1 | 08-11-2018 |
| | | US | 2019380685 A1 | 19-12-2019 |
| US 2023316642 A1 | 05-10-2023 | CN | 116097311 A | 09-05-2023 |
| | | EP | 4200812 A1 | 28-06-2023 |
| | | JP | 2023538578 A | 08-09-2023 |
| | | US | 2023316642 A1 | 05-10-2023 |
| | | WO | 2022038208 A1 | 24-02-2022 |
| US 2022005258 A1 | 06-01-2022 | CN | 112272850 A | 26-01-2021 |
| | | EP | 3553785 A1 | 16-10-2019 |
| | | EP | 3776572 A1 | 17-02-2021 |
| | | JP | 7278304 B2 | 19-05-2023 |
| | | JP | 2021520870 A | 26-08-2021 |
| | | US | 2022005258 A1 | 06-01-2022 |
| | | WO | 2019197203 A1 | 17-10-2019 |
| US 2023073704 A1 | 09-03-2023 | CN | 115279273 A | 01-11-2022 |
| | | EP | 4114270 A1 | 11-01-2023 |
| | | JP | 7278500 B2 | 19-05-2023 |
| | | JP | 2023505621 A | 09-02-2023 |
| | | US | 2023073704 A1 | 09-03-2023 |
| | | WO | 2021176030 A1 | 10-09-2021 |

EPO FORM P0459

For more details about this annex : see Official Journal of the European Patent Office, No. 12/82